Europäisches Patentamt

(19) European Patent Office

Office européen des brevets

(11) Publication number: **0 037 667**
**B1**

# EUROPEAN PATENT SPECIFICATION

(45) Date of publication of patent specification: **27.06.84**

(21) Application number: **81301213.5**

(22) Date of filing: **20.03.81**

(51) Int. Cl.³: **C 12 N 11/08,**
**C 12 P 19/02,**
**A 23 C 21/02, A 23 C 9/12**

(54) **Immobilized lactase, its preparation and use.**

(30) Priority: **04.04.80 JP 45044/80**

(43) Date of publication of application:
**14.10.81 Bulletin 81/41**

(45) Publication of the grant of the patent:
**27.06.84 Bulletin 84/26**

(84) Designated Contracting States:
**BE DE FR GB IT NL**

(56) References cited:
**DE - A - 2 818 086**
**FR - A - 2 198 953**
**FR - A - 2 381 059**
**GB - A - 1 557 944**
**US - A - 3 767 531**

(73) Proprietor: **SUMITOMO CHEMICAL COMPANY,**
**LIMITED**
**15 Kitahama 5-chome Higashi-ku**
**Osaka-shi Osaka-fu (JP)**

(72) Inventor: **Hirohara, Hideo**
**2-1-146, Kuwatacho Ibaraki-shi**
**Osaka (JP)**
Inventor: **Yamamoto, Hidefumi**
**26-506, Tamagawa 1-chome Takatsuki-shi**
**Osaka (JP)**
Inventor: **Kawano, Emiko**
**4-14, Noda 6-chome Fukushima-ku**
**Osaka (JP)**
Inventor: **Nabeshima, Shigeyasu**
**2-1-344, Kuwatacho Ibaraki-shi**
**Osaka (JP)**
Inventor: **Mitsuda, Satoshi**
**26-409, Tamagawa 1-chome Takatsuki-shi**
**Osaka (JP)**
Inventor: **Nagase, Tsuneyuki**
**12-11, Miyanokawahara 5-chome Takatsuki-shi**
**Osaka (JP)**

(74) Representative: **Myerscough, Philip Boyd et al,**
**J.A.Kemp & Co. 14, South Square Gray's Inn**
**London, WC1R 5EU (GB)**

Courier Press, Leamington Spa, England.

## Description

The present invention relates to a water-insoluble and enzymatically active immobilized lactase. More particularly, the invention pertains to a stable and water-insoluble immobilized lactase having a high lactose-hydrolyzing activity, which is prepared by immobilizing a lactase derived from *Aspergillus oryzae* on a macroporous phenol-formaldehyde based anion-exchange resin.

When an enzyme, which *per se* is a catalyst for a reaction in homogeneous aqueous solution, is immobilized, it becomes possible to be used repeatedly and continuously. In reflection of such usefulness of an immobilized enzyme in an industrial application, there have recently been developed a number of enzyme-immobilization techniques [C. R. Zaborsky: Immobilized Enzymes, C. R. C. Press, (1973); Ichiro Chihata, Immobilized Enzymes, Kodansha (1975)].

Hitherto known methods for producing immobilized enzymes may roughly be divided into the following four methods.

    (1) Adsorption Method
    (2) Covalent Attachment Method
    (3) Entrapment Method
    (4) Crosslinking Method

Since each method has both advantages and disadvantages over the others, it is hard to tell which one is superior to the others. Moreover, there has not yet been provided the enzyme-immobilization carrier and the enzyme-immobilization method which are applicable to any and all diverse enzymes. In practice, a suitable carrier and a method must individually be selected depending upon a particular purpose so that the excellent immobilized enzyme which serves the intended purpose may be obtained.

In addition thereto, attention should be paid to the fact that even the enzymes which are classified in the same category and given an identical enzyme number in accordance with the Report of the Commision on Enzymes of the International Union of Biochemistry, 1964, may be quite different from each other in molecular weight and enzymatical properties if the origins of the enzymes are different. In such a case, it is quite natural that the combination of a particular enzyme with a carrier and a method by which combination a very advantageous immobilized enzyme may be obtained varies depending upon the origins of the enzymes even if the enzymes are given the same enzyme number and the same name.

Lactase($\beta$-galactosidase; enzyme number 3. 2. 1. 23) is the enzyme which hydrolyzes lactose into glucose and galactose. It is known that animals, plants, bacteria, fungi and yeast produce lactase. Lactase is a typical example of the enzymes under the name of which there are many kinds of enzymes which are different from each other in their properties except one common property of hydrolyzing lactose into glucose and galactose. For example, of the enzymes originated from bacteria, the lactase derived from *Escherichia coli* (hereinafter "*E. coli*") is the intracellular enzyme of which molecular weight is in a range from $52 \times 10^4$ to $54 \times 10^4$, the optimum pH is 7.3 and Michaelis constant Km (which represents dissociation constant of enzyme-substrate complex) is $9.5 \times 10^{-4}$ M [substrate; ortho-nitrophenyl-$\beta$-D-galactopyranoside (ONPG)], and $7.5 \times 10^{-3}$ M (substrate; lactose).

On the other hand, the lactase derived from yeast such as *Sacchromyces fragilis* or *Saccharomyces lactis* is also the intracellular enzyme of which molecular weight is as low as $20 \times 10^4$, less than a half of that of the *E. coli*-originated lactase. The optimum pH of the lactase originated from the yeast is 6.5 to 7.0 and Km values (substrate; ONPG and lactose) are much different from those of the *E. coli*-originated lactase.

Moreover, the lactase originated from a fungus, *Aspergillus niger* is extracellular enzyme of which optimum pH is as low as 3.5 and whose molecular weight is in a range from $10 \times 10^4$ to $11 \times 10^4$. The lactase originated from a similar fungus, *Aspergillus oryzae* has many differences from the *Aspergillus niger*-originated enzymes including the optimum pH being 4.5 to 5.0 (C. D. Boyer: The Enzymes, the third edition, Vol. 7, 617—663).

Thus, it is hardly believed that the lactases whose origins are different from the others have the same chemical structure and properties and are the same compound. They have only one common property in that they all hydrolyze lactose into glucose and galactose or aryl- or alkyl-$\beta$-D-galactoside into an alcohol and galactose.

Accordingly, it may be said that the enzyme-immobilization carrier and the enzyme-immobilization method which are suited to a certain enzyme are not necessarily applicable to all other enzymes. For instance, U.S. Patent 3,767,531 discloses a method of immobilizing the lactase originated from *Aspergillus niger* on phenol-formaldehyde resin which has only phenolic hydroxyl groups and methylol groups but no other functional groups such as amino or substituted amino group or carboxymethyl groups. However, those carriers and method are not necessarily suitable to all kinds of the enzymes. This fact is clear from the following description: Using an immobilized lactase produced by adsorbing *Bacillus circulans*-originated lactase on Duolite® ES-762, a phenol-formaldehyde resin having phenolic hydroxyl groups and methylol groups but no other ion-exchange groups, followed by the treatment with glutaraldehyde, the study had been made to hydrolyze lactose in milk in a plug-flow reactor (column), but it was hard to retain the activity. The activity is decreased as fast as less than 6 days in the half-life, even when a lactose solution of 4.8% concentration is used as the substrate. [Report by Torii et al. page 38, Symposium of the Agricultural Chemical Society of Japan held in Tokyo, (April, 1979); Report by Matsuno, page 14, the 2nd lecture meeting of Enzyme Technological Studies. (1979)]. This is also demonstrated by Comparative Examples as shown hereinafter.

One major industrial application of lactase is for the hydrolysis of lactose in milk for a person who cannot tolerate lactose in milk, and one other application of lactase is the hydrolysis of lactose in whey. Accordingly, considerable attempts have been made in order to develop microorganism-originated lactases suitable for the said two major industrial applications and the method for immobilizing them [Immobilized Enzyme Technology, edited by H. H. Weetall and S. Suzuki, Plenum Press, New York (1975); Immobilized Enzymes in Food and Microbial Processes, edited by A. C. Olson and C. L. Cooney, Plenum Press, New York (1974)].

In particular, recent developments of techniques on membranes made it possible to separate whey into a high molecular weight protein part and a low molecular weight compound part comprising mainly lactose without difficulties. In view of this, a variety of studies on the hydrolysis of lactose with an immobilized lactase and an application of the product in the field of food industry have been made.

However, almost none of the studies has been put to practical use. The reason for this would be the fact that very few lactase are effective to both milk and acid whey and that there is a problem of product inhibition by galactose and the like. Among them, the followings would be the main two reasons:

1. Few lactases were stable, highly active to lactose and available at a reasonable price.
2. No available immobilized lactases were stable and highly active to lactose.

As previously discussed, an immobilized lactase is considered more important from the view point of an industrial application of lactase, but no single immobilization method is generally applicable to all kinds of lactases regardless of the origins of the lactase. In order to prepare an excellent immobilized lactase, it is necessary to carefully select the best combination of (1) a lactase, i.e. the kind of the lactase in terms of the origin, (2) a carrier and (3) an immobilization method. In other words, it is necessary to select the best lactase from the practical point of view, and to decide the most suitable carrier and immobilization method taking the manner of the industrial application of the resulting product into consideration. This, of course, is applicable to any other immobilized enzymes.

From this standpoint, we have developed new and excellent immobilization-carriers (as disclosed in, for example, published unexamined Japanese patent applications JP—A—67091/1979 and JP—A—119084/1979).

Moreover, there have been developed an immobilized lactase by means of adsorption-immobilization of *Aspergillus oryzae*-originated lactase on one of carriers developed by us, a macroporous phenol-formaldehyde based amphoteric ion-exchange resin having both anion- and cation-exchange groups, i.e. unsubstituted and substituted amino groups and carboxyl groups and having specific physical properties (Japanese patent application No. 103343/1979, published as JP—A—26189/1981 on 13 March 1981), and an immobilized lactase by means of covalent attachment through a glutaraldehyde treatment (Japanese patent application No. 127754/1979, corresponding to EP—A—0026672 published on 8 April 1981). The former immobilized by means of the adsorption-immobilization is fast in the immobilization of enzyme and has a high enzyme content per unit weight of the carrier and high activity, and the latter immobilized by means of covalent attachment is stable without causing elution of the enzyme even when a substrate contains a high concentration of salt. In addition thereto, due to the optimum pH peculiar to the *Aspergillus oryzae*-originated lactase, these immobilized lactases are effective within a pH range wider than those of the immobilized enzymes produced from yeast-originated lactase and *Aspergillus niger*-originated lactase, and effective to acid whey, whey permeate and milk. Thus, the lactase immobilized by means of covalent attachment of *Aspergillus oryzae*-originated lactase on the macroporous phenol-formaldehyde based amphoteric ion-exchange resin can be said to be extremely superior from industrial viewpoint. However, if some drawbacks must be pointed out, the preparation and conditioning of the carrier may be troublesome because of the ion-exchange resin to be used as the carrier being amphoteric.

The present inventors have earnestly studied to improve said drawbacks, and found that in case of an anion-exchange resin being used for the immobilization without the glutaraldehyde treatment, the content of immobilization enzyme per unit weight of the carrier is somewhat lower, and elution of the enzyme is easier, as compared with the macroporous phenol-formaldehyde based amphoteric ion-exchange resin being used for the immobilization, whereas an immobilized lactase which has a high activity per unit (i.e. specific activity) and which is so stable that no enzyme is eluted from the carrier, may be obtained when the immobilization is carried out through a glutaraldehyde treatment using a macroporous phenol-formaldehyde based anion-exchange resin having specified physical properties.

Accordingly, the present invention provides a water-insoluble, enzymatically-active immobilized lactase having:

(1) an optimum pH with respect to lactose of from 4.5 to 5.0,

(2) a Michaelis constant, Km, of $0.2\pm0.1$ mole/l (both at pH 4.5, 30°C and at pH 6.65, 30°C, substrate: lactoase), and

(3) an inhibition constant by galactose, Ki, of $(8\pm5)\times10^{-2}$ mole/l (pH 4.5, 30°C, substrate: lactose) and $(3\pm2)\times10^{-2}$ mole/l (pH 6.65, 30°C, substrate: lactose);

the immobilized lactase having been obtained by immobilizing a lactase obtained from a strain of *Aspergillus oryzae*, by means of covalent attachment, on a carrier of a macroporous phenol-formaldehyde based anion-exchange resin having a specific surface area of 5 m²/g or more, a total pore volume for pores having a pore diameter of 100 to 2000 Å of 0.2 cc/g or more, and an anion-exchange capacity due to amino groups and/or substituted amino groups of 1 meq/g or more; the lactase obtained from a strain of *Aspergillus oryzae* having:

(1) an optimum pH with respect to lactose of from 4.5 to 5.0,

(2) a Michaelis constant, Km, of $0.1\pm0.05$ mole/l (pH 4.5, 30°C, substrate: lactose) and $0.3\pm0.15$ mole/l (pH 6.65, 30°C, substrate: lactose), and

(3) an inhibition constant by galactose, Ki, of $(6\pm3)\times10^{-3}$ mole/l (pH 4.5, 30°C, substrate: lactose) and $(4\pm3)\times10^{-3}$ mole/l (pH 6.65, 30°C, substrate: lactose); and

the immobilization having been accomplished by adsorbing the lactase obtained from a strain of *Aspergillus oryzae* on the carrier of the macroporous phenol-formaldehyde based anion-exchange resin by immersing the carrier in an aqueous solution of pH 4.0 to 6.5 containing the lactase at from 5°C to 45°C and treating the carrier adsorbing the lactase with an aqueous solution of glutaraldehyde at pH 4.0 to 6.5 and at 5°C to 45°C.

The immobilized lactase of the present invention enables a simple and effective use of lactase in a continuous contact reaction of the substrate, lactose. The immobilized lactase produced by means of covalent attachment in accordance with the present invention has advantages from viewpoint of industrial preparation and has an activity and stability sufficiently high from standpoint of industrial utilisation.

The macroporous phenol-formaldehyde based anion-exchange resin usable in the present invention may be any one prepared in any method as far as the resin obtained has the above defined properties. The resin may be prepared by a known method, and some of the ion-exchange resin having the above defined properties are commercially available.

The shape of the resin may be granular or bead, and the size may be preferably in a range from 12 mesh (1410 $\mu$) to 60 mesh (250 $\mu$). It is not preferable to use a too large size of resin because the void volume is large in proportion to the size of resin, and, accordingly, the activity per volume becomes smaller. A too small size of the resin is not preferable either because the pressure drop is too large or the separation of the reaction solution from the immobilized enzyme is difficult.

The lactase used in the present invention is obtained from a strain belonging to *Aspergillus oryzae* as extracellular enzyme, and has the following characteristics:

1. Optimum pH and pH-dependence:

The optimum pH is pH 4.5 to pH 5.0 when the substrate is lactose. The ratio of the activity at pH 4.5 at 30°C to that of pH 6.65 at 30°C is 1:$0.45\pm0.05$.

2. Michaelis constant Km:

$Km\simeq0.10\pm0.05$ mole/l (pH 4.5, 30°C, substrate; lactose), and $Km\simeq0.3\pm0.15$ mole/l (pH 6.65, 30°C, substrate; lactose).

3. Inhibition constant Ki by galactose:

$Ki=(6\pm3)\times10^{-3}$ mole/l (pH 4.5, 30°C, substrate; lactose), and $Ki=(4\pm3)\times10^{-3}$ mole/l (pH 6.65, 30°C, substrate; lactose).

It is believed that the characteristics of the lactase are well defined by the above data, and some other properties are given below:

4. pH-stability:

When the lactase was immersed in a buffer solution in the various pH range at 40°C for one hour, the lactase was found to be stable and no decrease of the activity was found in the pH range of 4.0 to 7.5.

5. Temperature-stability:

When the lactase was immersed in a buffer solution of pH 4.5 and pH 6.5 at 50°C or lower for 30 minutes, the lactase did not lose its activity at all. The following table shows the residual activity of the enzyme after the enzyme was immersed at pH 4.5 and pH 6.5 at 55°C or higher for 30 minutes.

| Temperature (°C) | Residual activity (%) |
| --- | --- |
| 55 | 96—97 |
| 60 | 80 |
| 65 | 20 |

No difference between the results of the experiments at pH 4.5 and pH 6.5 was found.

6. Activation Energy, Ea:

Arrhenius plots [substrate; 13.3 (w/v)% of lactose solution, pH 4.5] have a slight curvature and Ea value at 30°C to 40°C is $9\pm3$ Kcal/mole.

The above values from 1 to 6 may, of course, include experimental errors permissible in the measurement of enzymatical reactions. As far as the above defined characteristics are satisfied, the process for producing the lactase is not particularly limited. The lactase is usually stored in the form of powder or solution. With respect to the activity, the enzyme of a low activity is meaningless from the gist of the present invention. Accordingly, it is preferable to use the enzyme powder of which enzymatical activity is no less than 15 ILU per mg of the un-immobilized enzyme powder, more preferably no less than 40 ILU/mg.

In the present specification, 1 ILU is defined as the amount of lactase which produces 1 $\mu$ mole per minute of glucose at pH 4.5 at 30°C when a 13.3 w/v% of lactase solution is used as the substrate. The quantity of glucose thus prepared is defined by using glucose oxidase-peroxidase-dye system. When the measurement of the activity (unit) is carried out at a different pH or temperature, the pH or the temperature will specifically be given in each case.

The immobilised lactase of the present invention is prepared by the following method:

The carrier is immersed in a solution of lactase in a buffer solution (pH 4.0 to pH 6.5 and at a temperature of 5°C to 45°C), whereby the lactase is adsorbed on the carrier. Then, the carrier adsorbing the lactase is treated with glutaraldehyde by immersing the carrier in an aqueous solution of glutaraldehyde adjusted to pH 4.0 to pH 6.5 and at a temperature of from 5°C to 45°C and stirring the mixture to give the immobilized lactase. Thereafter, unreacted glutaraldehyde is washed away. It is believed that glutaraldehyde cross-links between the enzyme and the carrier to immobilise the enzyme on the carrier with a covalent attachment. In fact, the enzyme is not eluted from the carrier even when a high salt concentration of the substrate solution is applied thereto.

In this immobilisation procedure, it should be noted that unadsorbed lactase is washed away after completion of the adsorption operation so that cross-linkings between the unadsorbed free enzymes are not formed. The treatment with glutaraldehyde after the adsorption is carried out within a pH range from 4.0 to 6.5, whereby the lactase adsorbed is not eluted during the glutaraldehyde treatment.

The adsorption and the reaction with glutaraldehyde are carried out at a temperature from 5°C to 45°C. It is preferable to carry out the adsorption at a temperature slightly higher than that of the treatment with glutaraldehyde, for example carrying out the adsorption at 30°C and the treatment with glutaraldehyde at 20°C, whereby the rate of adsorption becomes high and almost no elution of the enzyme occurs during the glutaraldehyde treatment.

Suitable concentrations of glutaraldehyde used in the present invention may be 0.1 to 5 w/v%, preferably about 0.2 to 2 w/v%.

The time necessary for the adsorption and the glutaraldehyde treatment depends on the temperature of each process, but is in general from about 0.5 to 20 hours for each process. In case where the adsorption and the treatment with glutaraldehyde are carried out at a temperature from about 15° to 40°C, 1 to 6 hours is long enough for each process.

The amount of powdery lactase to be immobilized is at most about 200 mg/g-dry carrier. Although both amount and percent of the immobilization enzyme are lower than those of the immobilized lactase disclosed in Japanese patent application No. 127754/1979, there is no problem in practice. The immobilized lactase in accordance with the present invention is rather superior to that in easiness in the preparation and availability of the carrier.

It is important to wash the immobilized lactase thoroughly with a buffer solution of a high salt concentration and water to remove all the lactase which is insufficiently bonded and hence is likely to be separated. The enzyme which is not separated by such a treatment is just the immobilized lactase of the present invention.

The enzymatical properties of the present immobilized lactase are given below.

1. Optimum pH and pH-dependency:
The optimum pH ranges from 4.5 to 5.0, when the substrate is lactose, and almost no change in the optimum pH values by the immobilization is observed. The ratio of the activity at pH 4.5 to the activity at pH 6.65 is 1:0.45±0.05.

2. Michaelis constant Km:
Km=0.2±0.1 mole/l (both pH 4.5, 30°C, substrate; lactose, and pH 6.65, 30°C, substrate; lactose)

3. Inhibition constant by galactose Ki:
Ki=(8±5)×10$^{-2}$ mole/l (pH 4.5, 30°C, substrate; lactose) and Ki=(3±2)×10$^{-2}$ mole/l (pH 6.65, 30°C, substrate; lactose).

Both values of Km and Ki for the immobilized enzyme tend to increase with increase in the concentration of the glutaraldehyde solution used for the treatment, and moreover, in the measurement thereof, it is considerably difficult to obtain the results with good reproducibility. Therefore, the above value given for Km and Ki should be understood taking this respect into consideration.

4. pH-stability:
When the immobilized enzyme was immersed in each buffer solution of different pH value at 40°C for one hour, the immobilized enzyme is stable, and no decrease of the activity is observed within a pH range from 2.0 to 7.0.

The immobilized lactase of the present invention is characterized by the above stated enzymatical properties, and some other practical characteristics to be noted are as described below:

## 1. The activity per unit weight (specific activity):

The activity per 1 g of the dry immobilized lactase (specific activity) is not particularly limited, but those having too low activity are worthless in view of the object of the present invention. (The dry immobilized lactase is hereinafter referred to as IML). After all, the preferred in practice are those having about 200 ILU/g-IML or more at pH 4.5 and 30°C. In the present invention, there are readily obtained those having an activity as high as about 500 to 1000 ILU/g-IML.

## 2. Resistance against chemicals:

When the immobilized lactase of the present invention is immersed in a 300 fold diluent of 10 w/v% benzalkonium chloride solution, a disinfectant, Osuban®, (sold by Takeda Chemical Industries, Ltd.), and allowed to stand for 3 months at 4°C, almost no decrease of the activity is observed. Thus, the present immobilized lactase is found to have a high resistance against chemicals.

## 3. Stability in a continuous reaction:

As illustrated in detail in Examples, hydrolysis of lactose is carried out as follows; The immobilized lactase of the present invention having, for example, a specific activity of 615 ILU/g-IML at pH 4.5 at 30°C is packed in a column equipped with a jacket, and a 7 w/v% lactose solution (pH 4.5) is continuously passed through the column kept at 40°C at a space velocity (SV) of 4.5 hr$^{-1}$ for 100 days. In this instance, the ratio of the hydrolysis is kept to be 98 to 100% and no decrease of the activity is observed. Thus, it is apparent that the present immobilized lactase is very stable in the continuous reaction.

The present invention is illustrated in more detail with reference to the following Examples.

The conditions of the measurements of the activity of the immobilized lactase of the present invention are explained first as follows:

## 1. Method for measuring the activity of the immobilized lactose:

The immobilized lactase (0.1 to 0.3 ml) is suspended in a 0.05 M acetate buffer solution (pH 4.5). To this solution, a solution of lactose in the same buffer solution as above is added to give a 13.3 w/v% lactose solution. The solution was shaken at 30°C for 15 minutes with a shaker (100 rpm, shaking width; 3.5 cm). After the removal of the immobilized lactase by filtration, the content of the glucose in the filtrate is determined using glucose oxidase, peroxidase and dye system. The amount of the enzyme which produces 1 $\mu$ mole of glucose in one minute is defined at 1 Unit (1 ILU). The pH level and the temperature of the measurement of the activity will be stated in each case in the specification.

## 2. Measurement of weight of the immobilized lactase and carrier:

The immobilized lactase or the carrier is spread out in a 2 mm or less thick layer and dried under reduced pressure (5 mmHg or less) at 50°C for not shorter than 8 hours until a constant weight is reached. Thereafter, the immobilized lactase or the carrier being tested is put in a desicator at a room temperature (18 to 25°C) for 1.5 hours or more and then the weight thereof is measured. This weight is considered to be the weight in the dry state. All of the weights indicated in this specification mean this dry weights.

Example 1
Preparation of immobilized lactase and its composition

A dry powder of lactase originated from *Aspergillus oryzae* (7.5 g) (produced by Shinnihon Kagaku Kogyo Co., the activity at pH 4.5 at 30°C: 58 ILU/mg, Km at pH 4.5 at 30°C: 0.10 mole/l) was dissolved in 375 ml of 0.05 M acetate buffer solution (pH 5.5). In this solution was immersed 50 g of a macroporous phenol-formaldehyde based anion-exchange resin of 250 to 840 $\mu$ in the particle diameter, 31 m$^2$/g in the specific surface area, 0.52 cc/g in the total volume of the macropores having a pore diameter of 100 to 2000 Å, and 7.5 meq/g in the anion-exchange capacity due to primary amino groups and secondary amino groups (commercially available Duolite® A-7, produced by Diamond Shamrock). The resulting mixture was stirred at 30°C for 4 hours at about 150 rpm to perform adsorption procedure. Thereafter, the product was washed thoroughly with a 0.2 M acetate buffer solution (pH 5.5) and deionized water until no enzyme protein appeared in the washings. The amount of the enzyme adsorbed was calculated to be 103 mg/g-carrier from the protein content of the washings. Thus obtained carrier adsorbing the lactase was immersed in 375 ml of a 0.8% glutaraldehyde solution (adjusted to pH 4.5), and stirred for 3 hours at 150 rpm while maintaining the temperature at 20 to 21°C. The resulting resin was washed thoroughly with a 0.2 M acetate buffer solution and deionized water. The amount of the enzyme immobilized was calculated to be 102 mg/g-carrier. The activity of the immobilized lactase thus obtained was found to be 615 ILU/g-IML at pH 4.5 at 30°C.

Continuous hydrolysis of lactose

The present immobilized lactase (10 ml) was packed in a column (inner diameter 12 mm) equipped with a jacket. A 7 w/v% lactose solution adjusted to pH 4.5 with a 0.05 M acetate buffer solution was continuously passed through the column kept at 40°C for 100 days at a space velocity of 4.5 hr$^{-1}$. The rate of hydrolysis was calculated from the glucose content in the eluted solution and was found to be kept within 98 to 100% during the said 100 days. Thus, no decrease of the activity was observed. The immobilized lactase in the column was sterilized once every two weeks with a 500 fold diluent of a commercially available disinfection-detergent, Diazan® (sold by Asahi Garasu K.K.).

Continuous hydrolysis of lactose in skim milk

A column (inner diameter 14 mm) packed with 15 ml of the present immobilized lactase was placed in a room of 4°C, and a 12 w/v% skim milk solution (total sugar content: 5 w/v%, pH 6.65) was continuously passed through the column for 85 days at a space velocity of 0.75 hr$^{-1}$. Although the rate of the hydrolysis of lactose was considerably uneven immediately after starting the hydrolysis, the average rate thereof were found to be 72.5% and 70.4% for the first 5 days after the starting and the last 5 days from 80th day to 85th day, respectively. Thus, the decrease of the activity, if any, was found to be very slight. In this experiment, the immobilized lactase was washed once every 5 days in average with a 200 fold diluent of Diazan® to prevent putrefaction of the skim milk, which is likely to occur when passed through the column.

Continuous hydrolysis of lactose in whey

A column (inner diameter 12 mm) packed with 10 ml of the present immobilized lactase was placed in a room of 4°C. Whey powder (made in New Zealand) was made into a 7 w/v% solution which was 4.9% in a lactose content, adjusted to pH 4.4; freed from the undissolved by means of a centrifuge and incorporated with 120 ppm of n-propyl p-hydroxybenzoate to prevent putrefaction. The solution was passed through the column at a space velocity of 2.4 hr$^{-1}$. Average rate of the hydrolysis of lactose for 5 days after starting the hydrolysis was found to be 87%. After the hydrolysis had been carried out continuously for 120 days, the rate of hydrolysis was found to be 78%. This decrease is of no great importance in practice.

In this experiment, the immobilized lactase was sterilized once every two weeks with a 500 fold diluent of Diazan®.

Measurement of Km and Ki

Km and Ki values of the present immobilized lactase were calculated from Lineweaver-Burk plots using a solution of lactose as a substrate. Km value was found to be 0.21 mole/l at pH 4.5 at 30°C, and Ki value $6.7 \times 10^{-2}$ mole/l when a 0.15 M solution of galactose was added. While, Km value was 0.22 mole/l at pH 6.65 at 30°C, and Ki value $2.5 \times 10^{-2}$ mole/l when a 0.1 M solution of galactose was added.

Temperature stability of activity

The temperature stability of the activity of the present immobilized lactase was determined at pH 4.5 using a 13.3% solution of lactose as a substrate, whereby Arrhenius plots between 0°C and 50°C had a curvature slightly. Each activation energy at about 30° to 40°C and about 5°C was found to be $7 \pm 2$ Kcal/mole and $11 \pm 2$ Kcal/mole, respectively.

Example 2
Preparation of immobilized lactase and its composition

A dry powder of lactase originated from *Aspergillus oryzae* (2.3 g) (the activity and Km value at pH 4.5 at 30°C being 73 ILU/mg and 0.11 mole/l, respectively) was dissolved in 150 ml of 0.05 M acetate buffer solution (pH 5.5). In this solution was immersed 20 g of a macroporous phenol-formaldehyde based anion-exchange resin of 250 to 840 $\mu$ in the particle diameter, 68 m$^2$/g in the specific surface area, 0.56 cc/g in the total volume of micropores having a pore diameter of 100 to 2000 Å, and 4.4 meq/g in the anion-exchange capacity due to tertiary amino groups (commercially available Duolite® A-4, produced by Diamond Shamrock). The stirring of the mixture was continued at a liquid temperature of $35 \pm 2$°C for 3 hours at about 150 rpm to adsorb the lactase on the carrier. Successively, the product was washed thoroughly with a 0.3 M acetate buffer solution (pH 5.5) and deionized water, and then the resin immobilizing the lactase was immersed in 150 ml of a 1.0% solution of glutaraldehyde (adjusted to pH 4.5). The stirring of the mixture was continued at a liquid temperature of 20°C for 4 hours at 150 rpm to perform the glutaraldehyde treatment. The amount of the enzyme immobilized was calculated to be 74 mg/g-carrier. The activity of the immobilized lactase thus obtained was found to be 635 ILU/g-IML at pH 4.5 at 30°C.

Continuous hydrolysis of lactose

A column (inner diameter 12 mm) equipped with a jacket was packed with 10 ml of the present immobilized lactase. Lactose was dissolved in a 0.05 M acetate buffer solution containing 150 ppm of n-propyl p-hydroxybenzoate to make a 7 w/v% solution of lactose. The resulting solution was continuously passed through the above column under conditions of 30°C, pH 5.5 and a space velocity of 4.9 hr$^{-1}$. In the continuous hydrolysis for 88 days, decrease in the rate of hydrolysis was found to be only from 75% to $71 \pm 2$%. In this experiment, the immobilized lactase in the column was sterilized once every two weeks with a 300 fold diluent of Osuban®.

Elution of enzyme by a solution of high salt concentration

A 0.3 M phosphate buffer solution (pH 6.65) was passed through a jacket-equipped column packed with 10 ml of the present immobilized lactase at a column temperature of 40°C for a week at a space velocity of 5.0 hr$^{-1}$. The buffer solution has a pH and an electric conductivity similar to those of milk. The amount of the enzyme, protein, eluted during this time was only 2.8% based on the weight of the immobilized enzyme, and decrease of the activity was also only 3% (the activity found being 615 ILU/g-carrier). Taking possible experimental errors into consideration, it can be said that no decrease of the activity occurs.

Hydrolysis of lactose in whey

A column (inner diameter 14 mm) equipped with a jacket was packed with 8 ml of the present immobilized lactase. A 7 w/v% solution of whey powder (made in New Zealand) containing 150 ppm of n-propyl p-hydroxybenzoate was centrifuged at 5000 G to remove the undissolved. The supernatant (adjusted to pH 4.5), which was somewhat emulsified, as the substrate solution was passed through the column at a column temperature of 40°C for 8 weeks at a space velocity of 8.5 hr$^{-1}$. Both the substrate solution and the product solution were cooled to about 2°C in order to prevent the putrefaction to the most extent, and also the immobilized enzyme in the column was sterilized once a week with a 300 fold diluent of a disinfection-detergent, Osuban®. Just before the sterilization the activity was seemingly decreased, but it was recovered immediately after the sterilization. After all, the hydrolysis rate of lactose in the first week was found to be 86% in average, and in the 8th week 85%. Thus, no decrease was observed within this period of time.

Measurement of Km value

Using lactose as the substrate, Km value was measured at pH 4.5 at 30°C, and at pH 4.5 at 40°C, and calculated from Lineweaver-Burk plots to be 0.27 mole/l and 0.34 mole/l, respectively.

Example 3
Preparation of immobilized lactase and its composition

A dry powder of lactase originated from *Aspergillus oryzae* (3.0 g) (produced by Shinnihon Kagaku Kogyo Co., activity; 80 ILU/mg at pH 4.5 at 30°C, Km value; 0.10 mole/l at pH 4.5 at 30°C) was dissolved in 150 ml of a 0.05 M acetate buffer solution (pH 5.2). In this solution was immersed 20 g of a macroporous phenol-formaldehyde based anion-exchange resin of 250 to 1000 $\mu$ in the particle diameter, 95 m$^2$/g in the specific surface area, 0.66 cc/g in the total volume of the macropores having a pore diameter of 100 to 2000 Å, and 4.3 meq/g in the anion-exchange capacity due to secondary amino groups and tertiary amino groups (commercially available Duolite® S-37, produced by Diamond Shamrock), and the adsorption procedure was carried out at 30°C for 4 hours. After sufficient washing, the amount of the enzyme adsorbed was calculated to be 99 mg/g-carrier. Successively, the resin adsorbing the lactase was immersed in 150 ml of a 1.5% glutaraldehyde solution (adjusted to pH 4.5), and kept at 15°C for 4 hours. The amount of the lactase immobilized was found to be 97 mg/g-carrier, and the activity of the immobilized lactase was 680 ILU/g-IML at pH 4.5 at 30°C.

Optimum pH and pH-dependency of the activity

Using a 13.3 w/v% solution of lactose, the pH-dependency of the activity of the present immobilized lactase was measured at 30°C. The optimum pH range was from pH 4.5 to pH 5.0, and the ratio of the activity at pH 6.65 to that at pH 4.5 was 0.44.

Storage stability

A part of the present immobilized lactase was kept at 35°C under a reduced pressure until the water content reached 50%. The immobilized lactase so dried (which looked dry) was sealed in the air and kept in a thermostat of 30°C for 2 months. Thereafter, when measured at pH 4.5 at 30°C, the activity retained 630 ILU/g-IML. Thus, the storage stability was favorable.

Example 4
Preparation of immobilized lactase and its composition

The same dry powder of lactase as used in Example 2 (1.5 g) was dissolved in 75 ml of a 0.05 M acetate buffer solution (pH 5.2). In this solution was immersed 10 g of a macroporous phenol-formaldehyde based anion-exchange resin of 250 to 1000 $\mu$ in the particle diameter, 90 m$^2$/g of the specific surface area, 0.6 cc/g in the total volume of macropores having a pore diameter of 100 to 2000 Å, and 1.9 meq/g in the anion-exchange capacity due to quaternary ammonium groups (prepared by the reaction between a commercially available Duolite® S-30 resin produced by Diamond Shamrock and 3 - chloro - 2 - hydroxypropyltrimethylammonium chloride to introduce quaternary ammonium groups to the resin). The stirring of the mixture was continued at 150 rpm for 5 hours while keeping the liquid temperature to 25°C, to adsorb the lactase on the carrier. After washing thoroughly with a 0.3 M acetate buffer solution (pH 5.2) and deionized water, the carrier having the enzyme immobilized by adsorption was immersed in 75 ml of a 2.0% glutaraldehyde solution (adjusted to pH 4.4), and the stirring was continued for 4 hours at 150 rpm while keeping the liquid temperature to 18 to 20°C, then performing the glutaraldehyde treatment. The amount of the enzyme immobilized was found to be 89 mg/g-carrier, and the activity thereof was 630 ILU/g-IML at pH 4.5 at 30°C.

Resistance against chemicals

The present immobilized lactase divided into four parts each in the amount corresponding to about 1 g of the lactase was immersed respectively in a 200 fold diluent of Diazan®, a 200 fold diluent of Osuban®, a lactic acid solution of pH 2.5 and a lactic acid-sodium hydroxide solution of pH 3.0, and then allowed to stand at 4°C for one month. Thereafter, each activity was measured at pH 4.5 at 30°C, and found to be 605, 625, 620 and 600 ILU/g-IML, respectively. Thus, almost no decrease of the activity was observed.

Comparative Example 1

A dry powder of lactase originated from a yeast, *Saccharomyces (Kluyveromyces) lactis* was adsorbed at pH 6.65 on the same carrier as used in Example 1, and then treated with a 1.5% glutaraldehyde solution (adjusted to pH 6.65) to be immobilized by means of covalent attachment. Thereafter, the activity was measured at pH 6.65 similar to the pH of milk at 30°C using a 13.3 w/v% lactose solution as the substrate, and found to be only 1 ILU/g-IML or less. Thus, there was only prepared the immobilized lactase having almost no activity in a practical sense.

Comparative Example 2

Immobilization on a carrier having no amino groups and carboxyl groups

Example 1 was repeated, provided that the procedure was carried out on a 1/5 scale using a commercially available phenol-formaline resin, Duolite® ES-762 (produced by Diamond Shamrock), having no ion-exchange groups other than phenolic hydroxyl groups and methylol groups, as the carrier for immobilizing the enzyme. The amount of the enzyme immobilized was found to be 49 mg/g-carrier at pH 4.5 at 30°C, and the activity thereof was only 270 ILU/g-IML, which was a half of that in Example 1.

Comparative Example 3

The industrial grade of lactase originated from *Aspergillus niger* was adsorbed on the same carrier as in Example 1, and then treated with a 1.5% glutaraldehyde solution (adjusted to pH 4.5). The activity of the resulting immobilized lactase was less than 1 ILU/g-IML at pH 6.65 which is similar to the pH of milk, at 30°C, and 45 ILU/g-IML even at 4.0 at 30°C. Thus, this immobilized lactase had a very low activity and no value in a practical sense.

**Claims**

1. A water-insoluble, enzymatically-active immobilised lactase having:

(1) an optimum pH with respect to lactose of from 4.5 to 5.0,
(2) a Michaelis constant, Km, of $0.2\pm0.1$ mole/l (both at pH 4.5, 30°C and at pH 6.65, 30°C, substrate: lactose), and
(3) an inhibition constant by galactose, Ki, of $(8\pm5)\times10^{-2}$ mole/l (pH 4.5, 30°C, substrate: lactose) and $(3\pm2)\times10^{-2}$ mole/l (pH 6.65, 30°C, substrate: lactose);

the immobilised lactase having been obtained by immobilizing a lactase obtained from a strain of *Aspergillus oryzae,* by means of covalent attachment, on a carrier of a macroporous phenol-formaldehyde based anion-exchange resin having a specific surface area of 5 m²/g or more, a total pore volume for pores having a pore diameter of 100 to 2000 Å of 0.2 cc/g or more, and an anion-exchange capacity due to amino groups and/or substituted amino groups of 1 meq/g or more; the lactase obtained from a strain of *Aspergillus oryzae* having:

(1) an optimum pH with respect to lactose of from 4.5 to 5.0,
(2) a Michaelis constant, Km, of $0.1\pm0.05$ mole/l (pH 4.5, 30°C, substrate:lactose) and $0.3\pm0.15$ mole/l (pH 6.65, 30°C substrate: lactose), and
(3) an inhibition constant by galactose, Ki, of $(6\pm3)\times10^{-3}$ mole/l (pH 4.5, 30°C, substrate: lactose) and $(4\pm3)\times10^{-3}$ mole/l (pH 6.65, 30°C, substrate: lactose); and

the immobilisation having been accomplished by adsorbing the lactase obtained from a strain of *Aspergillus oryzae* on the carrier of the macroporous phenol-formaldehyde based anion-exchange resin by immersing the carrier in an aqueous solution of pH 4.0 to 6.5 containing the lactase at from 5°C to 45°C and treating the carrier adsorbing the lactase with an aqueous solution of glutaraldehyde at pH 4.0 to 6.5 and at 5°C to 45°C.

2. An immobilized lactase according to claim 1, wherein the activity per gram of the dry immobilised lactase is 200 ILU or more at pH 4.5 and 30°C.

3. An immobilised lactase according to claim 1 or 2, wherein the adsorption of the lactase on the carrier is carried out at a temperature higher than the temperature of the glutaraldehyde treatment.

4. A process for hydrolysing lactose, which process comprises contacting lactose with an immobilised lactase as claimed in any one of claims 1 to 3.

**Patentansprüche**

1. Wasserunlösliche, enzymatisch wirksame, immobilisierte Lactase, mit

(1) einem optimalen pH-Bereich gegenüber Lactose von 4,5 bis 5,0,
(2) einer Michaelis-Konstanten Km von $0,2\pm0,1$ Mole/l bei sowohl pH 4,5 und 30°C als auch pH 6,65 und 30°C und Lactose als Substrat, und
(3) einer Inhibierungskonstante durch Galactose Ki von $(8\pm5)\times10^{-2}$ Mole/l bei einem pH von 4,5 und 30°C und Lactose als Substrat und $(3\pm2)\times10^{-2}$ Mole/l bei einem pH von 6,65 und 30°C und Lactose als Substrat,

wobei die immobilisierte Lactase erhalten ist durch Immobilisierung einer vom Stamm Aspergillus oryzae erhaltenen Lactase über eine kovalente Bindung an einem Trägermaterial aus einem makroporösen Anionenaustauscherharz auf Basis von Phenolformaldehyd mit einer spezifischen Oberfläche von 5 m$^2$/g oder mehr, einem Gesamtporenvolumen für Poren mit einem Porendurchmesser von 100 bis 2000 Å von 0,2 cm$^3$/g oder mehr und einer Anionenaustauscherkapazität aufgrund der Aminogruppen und/oder substituierten Aminogruppen von 1 meq/g oder mehr, und wobei die vom Stamm Aspergillus oryzae erhaltene Lactase

(1) einen optimalen pH-Bereich gegenüber Lactose von 4,5 bis 5,0,

(2) eine Michaelis-Konstante Km von 0,1±0,05 Mole/l bei einem pH von 4,5 und 30°C und Lactose als Substrat und 0,3±0,15 Mole/l bei einem pH von 6,65 und 30°C und Lactose als Substrat, und

(3) eine Inhibierungskonstante durch Galactose Ki von (6±3)×10$^{-3}$ Mole/l bei einem pH von 4,5 und 30°C und Lactose als Substrat und (4±3)×10$^{-3}$ Mole/l bei einem pH von 6,65 und 30°C und Lactose als Substrat aufweist, und

die Immobilisierung erreicht wurde durch Adsorption der vom Stamm Aspergillus oryzae erhaltenen Lactase an dem makroporösen Anionenaustauscherharz auf Phenolformaldehydbasis als Trägermaterial durch Tränken des Trägermaterials in einer die Lactase enthaltenden wäßrigen Lösung bei pH 4,0 bis 6,5 und 5 bis 45°C und Behandlung des Trägermaterials, an dem die Lactase adsorbiert ist, mit einer wäßrigen Glutaraldehydlösung bei pH 4,0 bis 6,5 und bei 5 bis 45°C.

2. Immobilisierte Lactase nach Anspruch 1, dadurch gekennzeichnet, daß die Wirksamkeit pro Gramm der trockenen, immobilisierten Lactase bei pH 4,5 und 30°C 200 ILU oder mehr ist.

3. Immobilisierte Lactase nach Anspruch 1 oder 2, dadurch gekennzeichnet, daß die Adsorption der Lactase an dem Trägermaterial durchgeführt wird bei einer Temperatur, die höher ist als die Temperatur der Glutaraldehydbehandlung.

4. Verfahren für die Hydrolyse von Lactose, dadurch gekennzeichnet, daß man Lactose mit einer immobilisierten Lactase gemäß einem der Ansprüche 1 bis 3 behandelt.

## Revendications

1. Lactase immobilisée, insoluble dans l'eau, et active du point du point de vue enzymatique ayant:

(1) un pH optimum par rapport au lactose de 4,5 à 5,0,

(2) une constante de Michaelis Km, de 0,2±0,1 mole/l (à la fois à pH 4,5, 30°C et à pH 6,65, 30°C, substrat: lactose), et

(3) une constante d'inhibition par le galactose Ki, de (8±5)×10$^{-2}$ moles/l (pH 4,5, 30°C, substrat: lactose) et de (3±2)×10$^{-2}$ moles/l (pH 6,65, 30°C, substrat: lactose);

la lactase immobilisée ayant été obtenue en immobilisant une lactase obtenue d'une souche d'Aspergillus oryzae, au moyen d'une liaison covalente, sur un support en une résine macroporeuse d'échange d'anions à base de phénolformaldéhyde, ayant une surface spécifique de 5 m$^2$/g ou supérieure à cette valeur, un volume total de pores pour des pores ayant un diamètre de pores de 100 à 2000 Å de 0,2 cm$^3$/g, ou supérieur à cette valeur, et une capacité d'échange d'anions due à des groupes amino et/ou à des groupes amino substitués de 1 meq/g ou supérieure à cette valeur; la lactase obtenue à partir d'une souche d'Aspergillus oryzae ayant:

(1) un Ph optimum par rapport au lactose de 4,5 à 5,0,

(2) une constante de Michaelis Km de 0,1±0,05 mole/l (pH 4,5, 30°C, substrat: lactose) et de 0,3±0,15 mole/l (pH 6,65, 30°C, substrat: lactose), et

(3) une constante d'inhibition par le galactose Ki de (6±3)×10$^{-3}$ mole/l (pH 4,5, 30°C, substrat: lactose) et de (4±3)×10$^{-3}$ mole/l (pH 6,65, 30°C, substrat: lactose); et

l'immobilisation ayant été effectuée en adsorbant la lactase obtenue à partir d'une souche d'Aspergillus oryzae sur le support de la résine macroporeuse d'échange d'anions à base de phénol-formaldéhyde en trempant le support dans une solution aqueuse de pH 4,0 à 6,5 contenant la lactase entre 5°C et 45°C et en traitant le support adsorbant la lactase par une solution aqueuse de glutaraldéhyde à un pH de 4,0 à 6,5 et entre 5°C et 45°C.

2. Lactase immobilisée suivant la revendication 1, dans laquelle l'activité par gramme de la lactase sèche immobilisée est de 200 ULI ou supérieure à cette valeur à un pH de 4,5 et à 30°C.

3. Lactase immobilisée suivant la revendication 1 ou 2, dans laquelle l'adsorption de la lactase sur le support est effectuée à une température supérieure à la température du traitement par le glutaraldéhyde.

4. Procédé pour hydrolyser du lactose qui consiste à mettre du lactose en contact avec une lactase immobilisée, tel que revendiqué suivant l'une des revendications 1 à 3.